(19) Europäisches Patentamt
European Patent Office
Office européen des brevets

(11) EP 3 395 807 B1

(12) EUROPEAN PATENT SPECIFICATION

(45) Date of publication and mention of the grant of the patent:
**14.10.2020 Bulletin 2020/42**

(21) Application number: **16879247.1**

(22) Date of filing: **13.12.2016**

(51) Int Cl.:
***C07D 309/38*** *(2006.01)* ***A61K 8/49*** *(2006.01)*
***A61Q 19/02*** *(2006.01)*

(86) International application number:
**PCT/KR2016/014592**

(87) International publication number:
**WO 2017/111380 (29.06.2017 Gazette 2017/26)**

(54) **SALICYLIC ACID DERIVATIVE, METHOD FOR PREPARING SAME, AND COSMETIC COMPOSITION FOR WHITENING COMPRISING SAME**

SALICYLSÄUREDERIVAT, VERFAHREN ZUR HERSTELLUNG DAVON UND KOSMETISCHE ZUSAMMENSETZUNG ZUM BLEICHEN DAMIT

DÉRIVÉ D'ACIDE SALICYLIQUE, SON PROCÉDÉ DE PRÉPARATION, ET COMPOSITION COSMÉTIQUE DE BLANCHIMENT COMPRENANT LE DÉRIVÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2015 KR 20150185920**

(43) Date of publication of application:
**31.10.2018 Bulletin 2018/44**

(73) Proprietor: **Amorepacific Corporation**
**Seoul 04386 (KR)**

(72) Inventors:
- **RHO, Ho Sik**
  **Yongin-si**
  **Gyeonggi-do 17074 (KR)**
- **YOO, Jae Won**
  **Yongin-si**
  **Gyeonggi-do 17074 (KR)**
- **KIM, Yong Jin**
  **Yongin-si**
  **Gyeonggi-do 17074 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
**KR-A- 20000 017 297** **KR-A- 20040 102 354**
**KR-A- 20120 088 036** **KR-A- 20130 004 616**
**KR-B1- 960 015 406** **US-A- 5 523 421**

- **JUN-CHEOL CHO ET AL: "Depigmenting activities of kojic acid derivatives without tyrosinase inhibitory activities", BIOORGANIC & MEDICINAL CHEMISTRY LETTERS, PERGAMON, AMSTERDAM, NL, vol. 22, no. 12, 9 April 2012 (2012-04-09) , pages 4159-4162, XP028509356, ISSN: 0960-894X, DOI: 10.1016/J.BMCL.2012.04.046 [retrieved on 2012-04-16]**
- **CHO, JUN-CHEOL ET AL.: 'Depigmenting Activities of Kojic Acid Derivatives without Tyrosinase inhibitory Activities' BIOORGANIC & MEDICINAL CHEMISTRY LETTERS vol. 22, no. 12, 09 April 2012, pages 4159 - 4162, XP028509356 DOI: 10.1016/J.BMCL.2012.04.046**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 395 807 B1

## Description

[Technical Field]

The present invention relates to a novel salicylic acid derivative, a process for preparing the same, and a cosmetic composition for whitening comprising the same.

[Background Art]

Melanin is a major factor which causes a difference in skin color between races or hyperchromatism such as freckles or ephelides. Melanin existing in the outer skin layer of the skin plays a role in protecting proteins and genes in the skin by absorbing free radicals and the like in the body, while protecting the skin organs beneath the dermis by blocking ultraviolet light.

The enzyme that plays a most important role in the formation process of melanin is known as tyrosinase. Tyrosinases converts tyrosine to DOPA and dopaquinone and produce melanic pigments through their non-enzymatic oxidation reaction. The production of melanin is promoted by stress stimuli inside and outside of the skin such as ultraviolet rays or inflammation, and melanin is a stable substance that does not disappear until it is released to the outside through keratinization of the skin, even if the stress is disappeared.

Therefore, components that inhibit the formation of melanic pigment or components that remove melanic pigment already deposited on the keratin and the like are used as a raw material for skin whitening agents. Examples of whitening components that inhibit the formation of melanic pigment include arbutin, kojic acid, and ascorbic acid. The substance that removes the pigment-deposited keratin may be alpha hydroxy acid (AHA), butylated hydroxyanisole (BHA), retinoids and the like.

Salicylic acid derivatives have also been reported to exhibit efficacy of removing the keratin and efficacy of promoting cell division. For example, Korean Patent Registration No. 10-0293758 discloses that alkyl salicylic acid or alkoxy salicylic acid has a whitening effect, and Korean Patent Registration No. 10-1453808 discloses a skin whitening cosmetic comprising an alkoxy salicylic acid as an effective component.

Cho, J.C., Rho, H.S., Joo, Y.H., Lee, C.S., Lee, J., Ahn, S.M., Kim, J.E., Shin, S.S., Park, Y.H., Suh, K.D. and Park, S.N., 2012. Depigmenting activities of kojic acid derivatives without tyrosinase inhibitory activities. Bioorganic & medicinal chemistry letters, 22(12), pp.4159-4162, discloses benzoate ester derivatives of kojic acid with and without adamantane moiety.

US 5,523,421 A discloses a kojic acid derivative comprising two substituents of hydroxy groups on the benzene ring.

However, the cosmetic compositions for whitening containing the components as mentioned above have limited and insufficient whitening effect, and thus have a problem that consumer satisfaction is not high. Therefore, it is necessary to develop a new whitening component that shows an improved effect as compared to the existing whitening components.

[Prior Art Literature]

Korean Patent Registration No. 10-0293758, EXTERNAL PREPARATION FOR SKIN, and
Korean Patent Registration No. 10-1453808, WATER-IN-OIL-TYPE SKIN-WHITENING COSMETIC.

[Disclosure]

[Technical Problem]

The present inventors have studied various compounds in order to develop a new whitening component exhibiting excellent whitening effect and as a result, have completed the present invention.

The embodiments of the present invention are reflected in independent claims 1, 2, and 4.

The preferred embodiment of the present invention is reflected in dependent claim 3.

Therefore, it is an object of the present invention to provide a novel salicylic acid derivative.

In addition, it is another object of the present invention is to provide a method for producing a salicylic acid derivative.

In addition, it is another object of the present invention is to provide a cosmetic composition for whitening comprising the salicylic acid derivative.

[Technical Solution]

In order to achieve the above objects, the present invention provides a salicylic acid derivative represented by the following Chemical Formula 1:

[Chemical Formula 1]

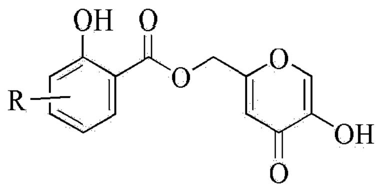

wherein R is a 5-methoxy group.

The salicylic acid derivative is 2-hydroxy-5-methoxy-benzoic acid 5-hydroxy-4-oxo-4H-pyran-2-ylmethyl ester.

The present invention also provides a method for preparing a salicylic acid derivative, characterized by preparing the compound of Chemical Formula 1 through reaction of the compound of Chemical Formula 2 with the compound of Chemical Formula 3, which is represented by the following Reaction Scheme 1:

[Reaction Scheme 1]

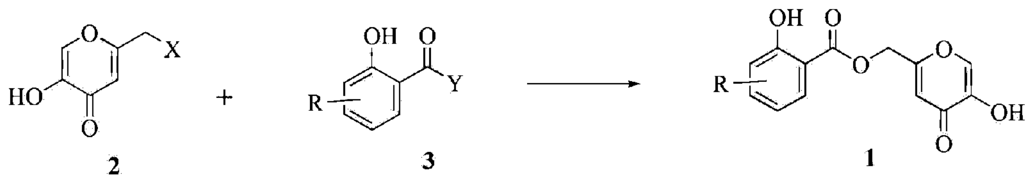

2 3 1

wherein R is a 5-methoxy group, X is Cl, and Y is ONa.

In addition, the present invention provides a cosmetic composition for whitening comprising the salicylic acid derivative represented by Chemical Formula 1 as an effective component.

[Advantageous Effects]

The salicylic acid derivative according to the present invention exhibits an improved skin whitening effect as compared with the existing whitening components. Specifically, the compound may exhibit a skin whitening effect by acting on tyrosinase, an enzyme that produces melanin, and thus inhibiting the production of melanin, which causes freckles or ephelides, and the compound can be formulated into a variety of cosmetic products.

[Best Mode]

Hereinafter, the present invention will be described in detail.

The present invention provides a salicylic acid derivative represented by the following Chemical Formula 1:

[Chemical Formula 1]

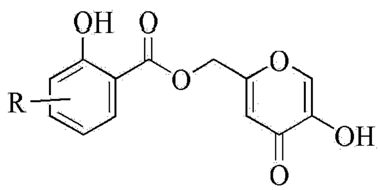

wherein R is a 5-methoxy group.

Comparative examples are provided where R is C1 to C4 alkyl group or a C1 to C4 alkoxy group other than 5-methoxy.

The C1 to C4 alkyl group referred to herein may be a linear or branched, saturated or unsaturated alkyl group.

The alkyl group may be, for example, a methyl group, an ethyl group, a propyl group, an isopropyl group, or a butyl group, preferably a methyl group.

In addition, the C1 to C4 alkoxy group referred to in the present specification may be a linear or branched, saturated or unsaturated alkoxy group. The alkoxy group may be, for example, a methoxy group, an ethoxy group, a propoxy group, an isopropoxy group, or a butoxy group, preferably a methoxy group other than 5-methoxy.

Non-limiting examples and comparative examples of the compounds represented by Chemical Formula 1 include the following compounds:

(1) 2-hydroxy-3-methyl-benzoic acid 5-hydroxy-4-oxo-4H-pyran-2-ylmethyl ester (Chemical Formula 2), as a comparative example

[Chemical Formula 2]

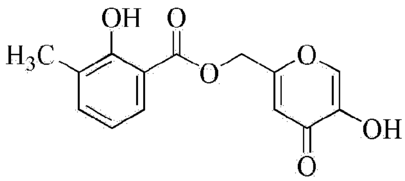

(2) 2-hydroxy-4-methyl-benzoic acid 5-hydroxy-4-oxo-4H-pyran-2-ylmethyl ester (Chemical Formula 3), as a comparative example

[Chemical Formula 3]

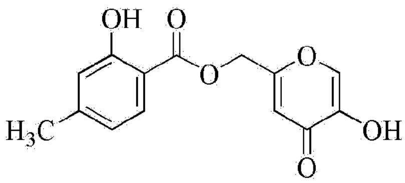

(3) 2-hydroxy-5-methyl-benzoic acid 5-hydroxy-4-oxo-4H-pyran-2-ylmethyl ester (Chemical Formula 4), as a comparative example

[Chemical Formula 4]

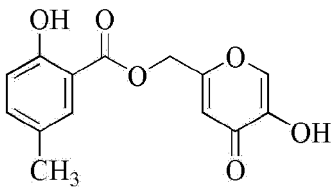

(4) 2-hydroxy-3-methoxy-benzoic acid 5-hydroxy-4-oxo-4H-pyran-2-ylmethyl ester (Chemical Formula 5), as a comparative example

[Chemical Formula 5]

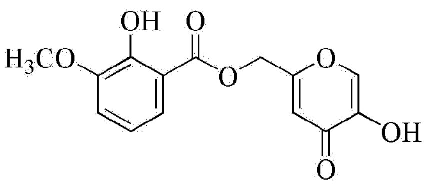

(5) 2-hydroxy-4-methoxy-benzoic acid 5-hydroxy-4-oxo-4H-pyran-2-ylmethyl ester (Chemical Formula 6), as a comparative example

[Chemical Formula 6]

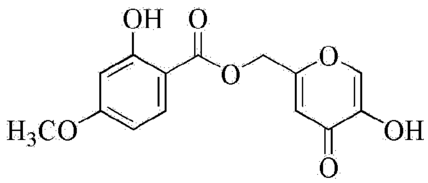

(6) 2-Hydroxy-5-methoxy-benzoic acid 5-hydroxy-4-oxo-4H-pyran-2-ylmethyl ester (Chemical Formula 7), as an example of the present invention,

[Chemical Formula 7]

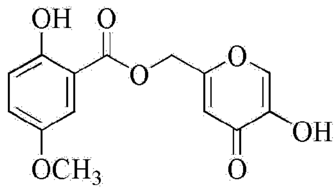

.

The compound of Chemical Formula 1 according to the present invention has excellent tyrosinase activity and thus can be used as a raw material for a cosmetic composition for whitening.

The compounds of Chemical Formula 1 of the present invention include all isomers unless otherwise specified.

Meanwhile, the present invention provides a method for preparing the compound of Chemical Formula 1 above.

Specifically, the compound of Chemical Formula 1 can be prepared by the esterification reaction of the compound of Chemical Formula 2 with the compound of Chemical Formula 3 as shown in the following Reaction Scheme 1:

[Reaction Scheme 1]

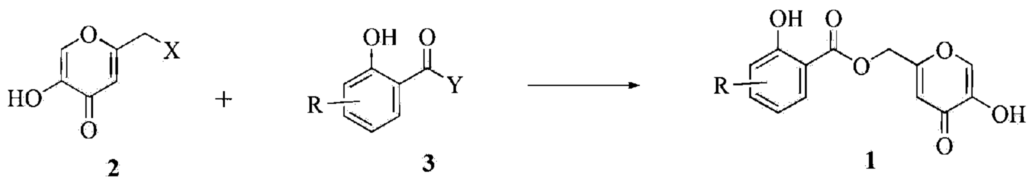

2 + 3 → 1

wherein,

R is a 5-methoxy group,
X and Y are OH, OM or a leaving group, provided that at least one of X and Y is a leaving group, and
M is Li, Na, or K.

Comparative examples are provided where R is C1 to C4 alkyl group or a C1 to C4 alkoxy group.

The leaving group may be, but is not limited to, halogen such as F, Cl, Br, and I, a C1-C4 alkoxy group, a substituted or unsubstituted (C1-C6) alkanesulfonyloxy group (e.g., a methanesulfonyloxy group, an ethanesulfonyloxy group or a trifluoromethanesulfonyloxy group), or a substituted or unsubstituted (C6-C12) arylsulfonyloxy group (e.g., a benzenesulfonyloxy group, p-toluenesulfonyloxy group, p-bromophenylsulfonyloxy group, p-nitrobenzenesulfonyloxy group)

In the above Reaction Scheme 1, the compound of Chemical Formula 2 and the compound of Chemical Formula 3, which are reaction materials, can be purchased, or can be directly prepared by methods known in the art.

Specifically, the alkali metal salt of the compound of Chemical Formula 2 or Chemical Formula 3 can be prepared by reacting commercially available kojic acid or salicylic acid with an alkali metal hydroxide (LiOH, NaOH, or KOH).

In addition, the compound of Chemical Formula 2 or Chemical Formula 3 including the leaving group can be respectively prepared by reacting commercially available kojic acid or salicylic acid with an appropriate reaction reagent according to methods known in the art.

According to an embodiment of the present invention, the salicylic acid derivative may be prepared by reacting kojic acid with thionyl chloride to produce 5-hydroxy-2-(chloromethyl)-4H-pyran-4-one and reacting it with the alkali metal

salt of alkyl or alkoxy salicylic acid. At this time, the alkali metal salt of the alkyl or alkoxy salicylic acid may be prepared by reacting alkyl or alkoxy salicylic acid with the inorganic base of LiOH, NaOH, or KOH in a polar solvent.

The reaction conditions of the reaction are not particularly limited and can be appropriately adjusted as necessary. Specifically, according to an embodiment of the present invention, the reaction temperature may be in the range of 10 to 200 °C, preferably 50 to 150 °C, and the reaction time may be in the range of 0.5 to 72 hours, preferably from 0.5 to 24 hours.

The solvent used in the reaction is not particularly limited, but the solvent is preferably an organic solvent, and specifically may be, but is not limited to, at least one selected from the group consisting of tetrahydrofuran (THF), acetone, di methyl formamide (DMF), acetonitrile, dimethyl sulfoxide (DMSO) and mixed solvents thereof.

The salicylic acid derivative of Chemical Formula 1 according to the present invention can be applied to various fields and is preferably used as an effective component for a cosmetic composition.

As shown in Experimental Example 1, the salicylic acid derivative of Chemical Formula 1 exhibits excellent skin whitening effect by significantly inhibiting the activity of tyrosinase, which is a major enzyme for the production of melanin, even at a concentration significantly lower than that of the known whitening components such as kojic acid, 3-methyl salicylic acid and 3-methoxy salicylic acid. Thus, the salicylic acid derivative of Chemical Formula 1 can be used as an effective component for cosmetic composition for whitening.

At this time, the content of the salicylic acid derivative of Chemical Formula 1 varies depending on the formulations, and as an example, may be 0.01 to 20 wt.%. If the effective component is contained in the above range, it is not only suitable for exhibiting the intended effect of the present invention but also can satisfy both stability and solubility of the composition, and it may also be appropriate to include in the above range in terms of cost-effectiveness.

The cosmetic composition may be prepared as any formulation conventionally prepared in the art, and for example, can be formulated, but is not limited thereto, into solution, suspension, emulsion, paste, gel, cream, lotion, powder, oil, powder foundation, emulsion foundation, wax foundation and spray, etc. More preferably, the cosmetic composition may be prepared as a formulations of sun cream, softening lotion, astringent lotion, nourishing lotion, nourishing cream, massage cream, essence, eye cream, pack, spray or powder.

In addition, the cosmetic composition according to the present invention may contain adjuvants conventionally used in the field of cosmetics or dermatology, such as fatty substances, organic solvent, solubilizers, thickeners, gelling agents, softener, antioxidants, suspending agents, stabilizers, foaming agent, perfumes, surfactants, water, ionic or nonionic type emulsifiers, fillers, metal ion sequestrants, chelating agents, preservatives, vitamin, blocking agents, wetting agents, essential oil, dye, pigment, hydrophilic or lipophilic active agents, lipid vesicle or any other ingredient commonly used in cosmetics. Such adjuvants are introduced in amounts commonly used in the field of cosmetics or dermatology.

When the formulation is a paste, a cream or a gel, animal oil, vegetable oil, wax, paraffin, starch, tragacanth, cellulose derivative, polyethylene glycol, silicone, bentonite, silica, talc, zinc oxide or the like may be used as a carrier component.

When the formulation is a powder or a spray, lactose, talc, silica, aluminum hydroxide, calcium silicate, or polyamide powder may be used as a carrier component, Particularly, in the case of the spray, it may further comprise propellants such as chlorofluorohydrocarbons, propane/butane or dimethyl ether.

When the formulation is a solution or an emulsion, a solvent, a solubilizing agent or an emulsifying agent is used as the carrier component, and examples thereof include water, ethanol, isopropanol, ethyl carbonate, ethyl acetate, benzyl alcohol, benzyl benzoate, propylene glycol, 1,3-butyl glycol oil, glycerol aliphatic ester, and fatty acid esters of polyethylene glycol or sorbitan.

When the formulation is a suspension, liquid diluents such as water, ethanol or propylene glycol, suspending agents such as ethoxylated isostearyl alcohol, polyoxyethylene sorbitol ester and polyoxyethylene sorbitan ester, microcrystalline cellulose, aluminum metahydroxide, bentonite, agar or tragacanth, etc., can be used as a carrier component.

Hereinafter, the present invention will be described in more detail by the following examples. It is to be understood, however, that the following examples are illustrative of the present invention only and are not intended to limit the scope of the present invention.

**Comparative Example 1: Preparation of 2-hydroxy-3-methyl-benzoic acid 5-hydroxy-4-oxo-4H-pyran-2-ylmethyl ester**

50g (0.35 mmol) of 5-Hydroxy-2-(hydroxymethyl)-4H-pyran-4-one was dissolved in 250ml of N, N-dimethylformamide, cooled in an iced water bath at 10 °C, and then 50 g (0.42 mol) of thionyl chloride was added dropwise for 30 minutes. After stirring at room temperature for 2 hours, the reaction solution was added to 2000 ml of ice water. The resulting solid was filtered, and the solid (filtered substance) was dissolved in 1000 ml of ethyl acetate. Magnesium sulfate and activated carbon were added, dried, discolored and filtered, and then the filtrate was concentrated and nucleic acid was added to obtain crystals. The crystals were dried in vacuo to obtain 39.5 g (70%) of the reaction product, 5-hydroxy-2-(chloromethyl)-4H-pyran-4-one as a yellow solid.

4.4 g (0.026 mol) of 3-methylsalicylic acid and 1.3 g (0.031 mol) of sodium hydroxide was dissolved in 40 ml of methanol. Methanol was distilled off and the residue was dissolved in 70 ml of N,N-dimethylformamide. 4.2 g (0.026 mol) of 5-hydroxy-2-(chloromethyl)-4H-pyran-4-one was added thereto and the mixture was heated and stirred in an oil bath at 110 °C for 2 hours. The solvent was distilled off and the residue was dissolved in 300 ml of ethyl acetate, and then the ethyl acetate solution was washed with 5% hydrochloric acid and distilled water, and dried and discolored by adding magnesium sulfate and activated carbon. Insolubles were filtered off and the filtrate was evaporated under reduced pressure to give 5.5 g (73% yield) of the reaction product as an off-white solid.

TLC (ethyl acetate : hexane = 1 : 1, v/v) $R_f$ = 0.45

$^1$H NMR (DMSO-$d_6$) δ 10.60 (s, 1H), 9.22 (s, 1H), 8.12 (s, 1H), 7.71 (d, 1H, J = 8.1 Hz), 7.48 (d, 1H, J = 8.1 Hz), 6.88 (m, 1H), 6.58 (s, 1H), 5.27 (s, 2H), 2.20 (s, 3H).

**Comparative Example 2: Preparation of 2-hydroxy-4-methyl-benzoic acid 5-hydroxy-4-oxo-4H-pyran-2-ylmethyl ester**

The target substance (5.3 g, 70%) was obtained as an off-white solid in substantially the same manner as in Example 1, except that 4-methylsalicylic acid was used instead of 3-methylsalicylic acid.

TLC (ethyl acetate : hexane = 1 : 1, v/v) $R_f$ = 0.44

$^1$H NMR (DMSO-$d_6$) δ 10.00 (s, 1H), 9.23 (s, 1H), 8.12 (s, 1H), 7.70 (d, 1H, J = 8.1 Hz), 6.82 (s, 1H), 6.80 (d, 1H, J = 8.1 Hz), 6.57 (s, 1H), 5.22 (s, 2H), 2.31 (s, 3H).

**Comparative Example 3: Preparation of 2-hydroxy-5-methyl-benzoic acid 5-hydroxy-4-oxo-4H-pyran-2-ylmethyl ester**

The target substance (5.9g, 78%) was obtained as an off-white solid in substantially the same manner as in Example 1, except that 5-methylsalicylic acid was used instead of 3-methylsalicylic acid.

TLC (ethyl acetate : hexane = 1 : 1, v/v) $R_f$ = 0.45

$^1$H NMR (DMSO-$d_6$) δ 10.10 (s, 1H), 9.24 (s, 1H), 8.12 (s, 1H), 7.60 (s, 1H), 7.36 (d, 1H, J = 8.1 Hz), 6.90 (d, 1H, J = 8.1 Hz), 6.59 (s, 1H), 5.21 (s, 2H), 2.24 (s, 3H).

**Comparative Example 4: Preparation of 2-hydroxy-3-methoxy-benzoic acid 5-hydroxy-4-oxo-4H-pyran-2-ylmethyl ester**

The target substance (4.9g, 65%) was obtained as an off-white solid in substantially the same manner as in Example 1, except that 3-methoxysalicylic acid was used instead of 3-methylsalicylic acid.

TLC (ethyl acetate : hexane = 1 : 1, v/v) $R_f$ = 0.43

$^1$H NMR (DMSO-$d_6$) δ 10.19 (s, 1H), 9.26 (s, 1H), 8.11 (s, 1H), 7.41 (d, 1H, J = 8.1 Hz), 7.20 (d, 1H, J = 8.1 Hz), 6.57 (s, 1H), 5.23 (s, 2H), 3.81 (s, 3H).

**Comparative Example 5: Preparation of 2-hydroxy-4-methoxy-benzoic acid 5-hydroxy-4-oxo-4H-pyran-2-ylmethyl ester**

The target substance (6.0g, 80%) was obtained as an off-white solid in substantially the same manner as in Example 1, except that 4-methoxysalicylic acid was used instead of 3-methylsalicylic acid.

TLC (ethyl acetate : hexane = 1 : 1, v/v) $R_f$ = 0.42

$^1$H NMR (DMSO-$d_6$) δ 10.60 (s, 1H), 9.20 (s, 1H), 8.06 (s, 1H), 7.78 (d, 1H, J = 8.1 Hz), 6.56 (s, 1H), 6.53 (s, 1H), 6.52 (s, 1H), 5.21 (s, 2H), 3.80 (s, 3H).

**Example 6: Preparation of 2-hydroxy-5-methoxy-benzoic acid 5-hydroxy-4-oxo-4H-pyran-2-ylmethyl ester**

The target substance (5.7g, 76%) was obtained as an off-white solid in substantially the same manner as in Example 1, except that 5-methoxysalicylic acid was used instead of 3-methylsalicylic acid.

TLC (ethyl acetate : hexane = 1 : 1, v/v) $R_f$ = 0.45

$^1$H NMR (DMSO-$d_6$) δ 9.92 (s, 1H), 9.22 (s, 1H), 8.08 (s, 1H), 7.24 (s, 1H), 7.18 (d, 1H, J = 8.1 Hz), 6.94 (d, 1H, J = 8.1 Hz), 6.60 (s, 1H), 5.29 (s, 2H), 3.72 (s, 3H).

**Experimental Example 1: Determination of the inhibitory effect on tyrosinase activity**

In order to investigate the tyrosinase activity inhibitory effect of the salicylic acid derivatives prepared in Examples

1 to 6, the following experiment was conducted.

Mushroom-derived tyrosinase and tyrosine were purchased from Sigma Chemical. Each sample was treated with 150 microliters of 0.1 M phosphate buffer (pH 6.5), 8 microliters of mushroom tyrosinase (2,100 units/ml, 0.05 M phosphate buffer, pH 6.5) and 36 microliters of 1.5 mM L-tyrosine by concentration. After the enzymatic reaction was carried out at 37 °C for 20 minutes, the activity of tyrosinase was measured by measuring the absorbance at 490 nm using a microplate reader (Bio-Rad 3550, Richmond, CA, U.S.A.). The inhibitory effect of tyrosinase of the salicylic acid derivatives prepared in Examples 1 to 6 was determined based on the following Equation (1).

[Equation 1]

Tyrosinase inhibition ratio (%) = 100 - ((Reaction absorbance of each sample/reaction absorbance of the control group) X 100)

The concentration of each test substance which decreased the activity of tyrosinase by 50% was determined by the above Equation, and the obtained results are shown in Table 1.

Table 1:

| The inhibitory effect on tyrosinase activity | |
|---|---|
| Test substance | Inhibition of tyrosinase activity ($IC_{50}$) |
| Kojic acid | 40.97 μM |
| 3-Methylsalicylic acid | > 200 μM |
| 3-Methoxysalicylic acid | > 200 μM |
| Compound of Comparative Example 1 | 3.20 μM |
| Compound of Comparative Example 2 | 1.51 μM |
| Compound of Comparative Example 3 | 5.30 μM |
| Compound of | 4.21 μM |
| Comparative Example 4 | |
| Compound of Comparative Example 5 | 4.20 μM |
| Compound of Example 6 | 3.29 μM |

As shown in Table 1, It can be seen that 3-methylsalicylic acid and 3-methoxysalicylic acid at a concentration of 200 μM or less do not exhibit the inhibitory effect on tyrosinase activity, and the compounds of Comparative Examples 1 to 5, especially working Example 6 exhibit a greatly enhanced inhibitory effect on tyrosinase activity as compared to starting compounds, methylsalicylic acid, methoxysalicylic acid, and kojic acid. Thus, it can be seen that the salicylic acid derivatives according to the present invention inhibit melanin production and have an excellent skin whitening effect.

Hereinafter, Formulation Examples of the present invention will be described. However, the cosmetic formulation containing the salicylic acid derivative according to the present invention is not limited to these examples.

**Formulation Example 1: Lotion**

The lotion was prepared according to the conventional method with the composition shown in the following table. [표2]

| Component | Content (wt.%) |
|---|---|
| Compound of Comparative Example 1 | 0.1 |
| Glycerine | 3.0 |

(continued)

| Component | Content (wt.%) |
|---|---|
| Butylene glycol | 2.0 |
| Propylene glycol | 2.0 |
| Carboxyvinyl polymer | 0.1 |
| PEG 12 nonylphenyl ether | 0.2 |
| Polysorbate 80 | 0.4 |
| Ethanol | 10.0 |
| Triethanolamine | 0.1 |
| preservative, Coloring, Perfume | Appropriate amount |
| Purified water | Remainder |

**Formulation Example 2: Nourishing cream**

The nourishing cream was prepared according to the conventional method with the composition shown in the following table.

Table 3:

| Component | Content (wt.%) |
|---|---|
| Compound of Comparative Example 1 | 2.0 |
| Polysorbate 60 | 1.5 |
| Sorbitan sesquioleate | 0.5 |
| PEG 60 Hydrogenated castor oil | 2.0 |
| Liquid paraffin | 10. |
| Squalane | 5.0 |
| Caprylic/capric triglyceride | 5.0 |
| Glycerine | 5.0 |
| Butylene glycol | 3.0 |
| Propylene glycol | 3.0 |
| Triethanolamine | 0.2 |
| Preservative, Coloring, Perfume | Appropriate amount |
| Purified water | Remainder |

**Formulation Example 3: Massage cream**

The massage cream was prepared according to the conventional method with the composition shown in the following table.

Table 4:

| Component | Content (wt.%) |
|---|---|
| Compound of Comparative Example 1 | 1.0 |
| Beewax | 10.0 |
| Polysorbate 60 | 1.5 |

(continued)

| Component | Content (wt.%) |
|---|---|
| PEG 60 Hydrogenated castor oil | 2.0 |
| Sorbitan sesquioleate | 0.8 |
| Liquid paraffin | 40.0 |
| Squalane | 5.0 |
| Caprylic/capric triglyceride | 4.0 |
| Glycerine | 5.0 |
| Butylene glycol | 3.0 |
| Propylene glycol | 3.0 |
| Triethanolamine | 0.2 |
| Preservative, Coloring, Perfume | Appropriate amount |
| Purified water | Remainder |

**Formulation Example 4: Pack**

The pack was prepared according to the conventional method with the composition shown in the following table.

Table 5:

| Component | Content (wt.%) |
|---|---|
| Compound of Comparative Example 1 | 0.2 |
| Polyvinyl alcohol | 13.0 |
| Sodium carboxymethylcellulose | 0.2 |
| Glycerine | 5.0 |
| Allantoin | 0.1 |
| Ethanol | 6.0 |
| PEG 12 nonylphenyl ether | 0.3 |
| polysorbate 60 | 0.3 |
| Preservative, Coloring, Perfume | Appropriate amount |
| Purified water | Remainder |

## Claims

1. A salicylic acid derivative represented by the following Chemical Formula 1:

[Chemical Formula 1]

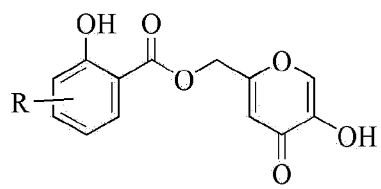

wherein R is 5-methoxy.

2. A method for preparing a salicylic acid derivative, **characterized by** reacting the compound of Chemical Formula 2 with the compound of Chemical Formula 3 to produce the compound of Chemical Formula 1 as shown in the following Reaction Scheme 1:

[Reaction Scheme 1]

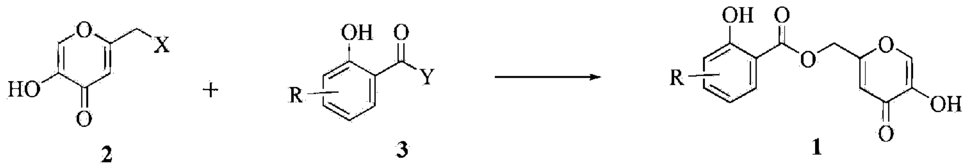

2 3 1

wherein,

R is 5-methoxy,
X and Y are OH, OM or a leaving group, provided that at least one of X and Y is the leaving group, and
M is Li, Na, or K.

3. The method for preparing the salicylic acid derivative according to claim 2, wherein X is Cl, and Y is ONa.

4. A cosmetic composition for whitening comprising the salicylic acid derivative according to claim 1 as an effective component.

**Patentansprüche**

1. Salicylsäurederivat, dargestellt durch die folgende chemische Formel 1:

[chemische Formel 1]

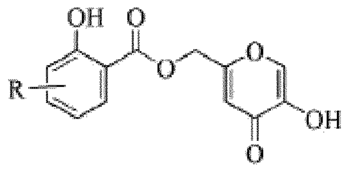

wobei R = 5-Methoxy ist.

2. Verfahren zur Herstellung eines Salicylsäurederivats, **dadurch gekennzeichnet, dass** man die Verbindung der chemischen Formel 2 mit der Verbindung der chemischen Formel 3 umsetzt, wobei die Verbindung der chemischen Formel 1 entsteht, wie in dem folgenden Reaktionsschema 1 gezeigt ist:

[Reaktionsschema 1]

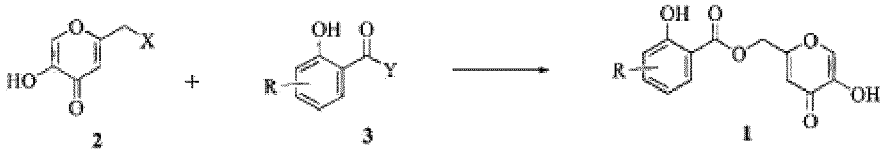

2 3 1

wobei

R = 5-Methoxy ist,

X und Y = OH, OM oder eine Abgangsgruppe sind, mit der Maßgabe, dass wenigstens eines von X und Y die Abgangsgruppe ist, und
M = Li, Na oder K ist.

3. Verfahren zur Herstellung des Salicylsäurederivats gemäß Anspruch 2, wobei X = Cl ist und Y = ONa ist.

4. Kosmetikzusammensetzung zum Weißmachen, die das Salicylsäurederivat gemäß Anspruch 1 als Wirkstoff umfasst.

**Revendications**

1. Dérivé d'acide salicylique représenté par la formule chimique 1 suivante :

[formule chimique 1]

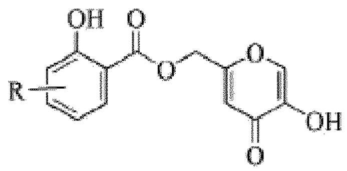

où R est 5-méthoxy.

2. Procédé pour préparer un dérivé d'acide salicylique, **caractérisé en ce que** l'on fait réagir le composé répondant à la formule chimique 2 avec le composé répondant à la formule chimique 3 pour produire le composé répondant à la formule chimique 1, comme on peut voir dans le schéma réactionnel 1 :

[schéma réactionnel 1]

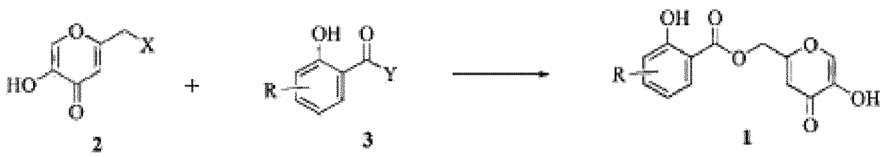

où

R est 5-méthoxy,
X et Y sont OH, OM ou un groupe partant, à condition qu'au moins un parmi X et Y est le groupe partant, et
M est Li, Na ou K.

3. Procédé pour préparer un dérivé d'acide salicylique selon la revendication 2, dans lequel X est Cl, et Y est ONa.

4. Composition cosmétique pour blanchiment, comprenant le dérivé d'acide salicylique selon la revendication 1 comme principe actif.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 100293758
- KR 101453808
- US 5523421 A


### Non-patent literature cited in the description


- **CHO, J.C. ; RHO, H.S. ; JOO, Y.H. ; LEE, C.S. ; LEE, J. ; AHN, S.M. ; KIM, J.E. ; SHIN, S.S. ; PARK, Y.H. ; SUH, K.D.** Depigmenting activities of kojic acid derivatives without tyrosinase inhibitory activities. *Bioorganic & medicinal chemistry letters,* 2012, vol. 22 (12), 4159-4162